# EUROPEAN PATENT APPLICATION

(11) **EP 3 511 010 A1**
(43) Date of publication of application: **17.07.2019**
(21) Application number: 17848172.7
(22) Date of filing: 08.09.2017
(51) Int. Cl.: A61K 35/60, A61K 9/107

(54) **FISH ODOR-FREE FISH OIL COMPOSITION AND PREPARATION METHOD THEREFOR**

(30) Priority: 09.09.2016 US 201662385606 P
(71) Applicant: TCI Co., Ltd., Taipei 11494 (TW)
(72) Inventor: LIN, Yung-Hsiang, Taipei 11494 (TW); KAO, Chen-I, Taipei 11494 (TW)
(74) Representative: Scholz, Volker
(86) International application number: PCT/CN2017/101107
(87) International publication number: WO 2018/045999

(57) **Abstract**

A fish odor-free fish oil composition includes a colloidal raw material and an oily raw material containing fish oil. A preparation method for the fish odor-free fish oil composition includes the following steps: (a) mixing and stirring the colloidal raw material with water and warming to 80 to 85 °C, continuing for 5 to 30 minutes, then cooling to 48 to 53 °C; and (b) stirring the oily raw material containing fish oil and emulsifying in an emulsification tank jointly with the colloidal mixture formed in step (a).

## Description

### CROSS-REFERENCE TO RELATED APPLICATION

This application claims priority of U.S. patent application No. 62/385,606, filed on September 9, 2016, the content of which is incorporated herein in its entirety by reference.

### BACKGROUND OF THE INVENTION

### 1. Field of the Invention

The present invention relates to a fish oil composition, particularly to a fish odor-free fish oil composition.

### 2. The Prior Art

Fish oil is a fat that is taken from fat-rich fish. For example, salmon, mackerel, herring or sardine are oil-rich fish. Fish oil contains omega-3 fatty acids, which are mainly two kinds of unsaturated fatty acids: eicosa pentanoic acid (EPA) and docosahexaenoic acid (DHA). Eicosa pentanoic acid and docosahexaenoic acid are important fatty acids that form phospholipids and cholesterol esters, having many effects, such as lowering high cholesterol, lowering breast cancer risk, improving Alzheimer's disease and Parkinson's disease, even can help to improve mental illnesses such as depression and anxiety.

In order to supplement EPA and DHA, many people would take supplements containing fish oil directly, but most of the fish oil supplements would have a fish odor, which causes most consumers to feel sick and nauseated. The fish odor is from trimethylamine produced by the metabolism of fish meat protein and spoilage. Therefore, the intensity of fish odor is closely related to the concentration of trimethylamine. Therefore, many people reject fish oil because they cannot accept the fish odor. Commercially available products reduce the fish odor by many methods, or improve the coating methods of fish oil to allow consumers to swallow a little in the abdomen, but there is still fish odor after swallowing in the abdomen, because an unpleasant smell is emitted from the mouth; or chemical additives are used to cover the fish odor of fish oil, but excessive intake of the chemical additives is harmful to the human body. Therefore, there is currently no method on the market that can effectively suppress or neutralize the fish odor.

### SUMMARY OF THE INVENTION

A primary objective of the present invention is to provide a fish odor-free fish oil composition, comprising: a colloidal raw material and an oily raw material comprising fish oil.

Preferably, the colloidal raw material is in an amount of 5% to 9% by weight, and the fish oil is in an amount of 5% to 20% by weight.

Preferably, the oily raw material further comprises a flavor in an amount of 0.5% to 5% by weight.

Preferably, the colloidal raw material comprises at least three components selected from the group consisting of: xanthan gum, guar gum, gelatin, sodium caseinate, arabic gum, sodium alginate, β-cyclodextrin, sodium carboxymethyl cellulose, Curdlan gum, gelatinized starch, starch acetate, distarch phosphate, phosphorylated distarch phosphate, and methyl cellulose.

Preferably, the flavor is selected from the group consisting of: lemon, mango, peach, orange, passion fruit, grapefruit, and combinations thereof.

Preferably, the oily raw material further comprises an ingredient selected from the group consisting of: vitamin E, xylitol, carotene, citric acid, potassium sorbate, sucralose, fruit juice, water, and combinations thereof.

Another objective of the present invention is to provide a method for the preparation of a fish odor-free fish oil composition, comprising the following steps: (a) mixing and stirring a colloidal raw material with water and warming to 80 to 85 °C, continuing for 5 to 30 minutes, then cooling to 48 to 53 °C to form a colloidal mixture; and (b) stirring an oily raw material comprising fish oil and emulsifying in an emulsification tank jointly with the colloidal mixture formed in step (a) to obtain the fish odor-free fish oil composition.

Preferably, the stirring in the steps (a) and (b) of the preparation method is uniformly mixed by a high pressure homogenizer at a pressure of 200 to 400 bar.

Accordingly, the present invention provides a fish odor-free fish oil composition comprising a colloidal raw material and an oily raw material comprising fish oil to overcome the disadvantages of a commercially available chemical additive or a fake fish oil that is harm to human health for covering the fish odor of fish oil; or providing fish oil supplements that reduce the content of omega-3 fatty acids to below 20% in order to reduce the fish odor of fish oil. Therefore, the fish odor-free fish oil composition of the present invention is a nutritional supplement which can continuously be ingested by the consumers without odor, thereby achieving the effects on maintaining elasticity of the artery, lowering cholesterol and triglyceride contents, reducing the incidence of stroke and myocardial infarction, and slowing vision and mental aging.

### BRIEF DESCRIPTION OF THE DRAWINGS

The following drawings form part of the present specification and are included here to further demonstrate some aspects of the present invention, which can be better understood by reference to one or more of these drawings, in combination with the detailed description of the embodiments presented herein.
FIG. 1 is a flow chart showing a method for the preparation of a fish odor-free fish oil composition of the present invention.
FIG. 2 is the result of gas chromatography analysis of the fish oil capsule products from the DSM Nutritional Products Canada Inc.
FIG. 3 is the result of gas chromatography analysis of the fish odor-free fish oil composition of the present invention.

### Description of the reference numerals in the drawings:

101, 102: Step number of the preparation method of the present invention.

### DETAILED DESCRIPTION OF THE PREFERRED EMBODIMENT

In the following detailed description of the embodiments of the present invention, reference is made to the accompanying drawings, which are shown to illustrate the specific embodiments in which the present disclosure may be practiced. These embodiments are provided to enable those skilled in the art to practice the present disclosure. It is understood that other embodiments may be used and that changes can be made to the embodiments without departing from the scope of the present invention. The following description is therefore not to be considered as limiting the scope of the present invention.

As used herein, the data provided represent experimental values that can vary within a range of ±20%, preferably within ±10%, and most preferably within ±5%.

Fish oil is the most important health food in all health foods, because fish oil acts on the human body's metabolism, mainly cardiovascular maintenance, activation of brain cells, regulation of hormones, vision improvement, anti-inflammation of skin, and promoting fetal development to make infants smarter. Therefore, the fish odor-free fish oil composition of the present invention can enhance the consumer's acceptance of fish oil supplements for the purpose of promoting good health.

### Example 1

### Preparation Method of Fish Odor-Free Fish Oil Composition of Present Invention

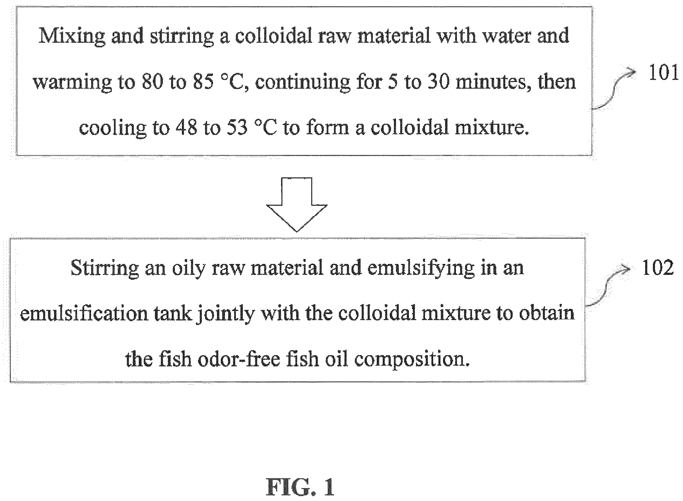

Referring to FIG. 1, which is a flow chart showing a method for preparing the fish odor-free fish oil composition of the present invention. The method comprises the following steps.

Step 101: Mixing and stirring a colloidal raw material with reverse osmosis (RO) water at 70±5 °C and warming to 80 to 85 °C, continuing for 5 minutes, then cooling to 48 to 53 °C to form a colloidal mixture. The colloidal raw material comprises xanthan gum and guar gum in a ratio of about 4:1.

Step 102: Stirring an oily raw material comprising fish oil, lemon flavor, and vitamin E at room temperature for 10 minutes, and emulsifying in an emulsification tank jointly with the colloidal mixture formed in Step 101 to obtain the fish odor-free fish oil composition of the present invention.

According to an embodiment of the present invention, the colloidal raw material is in an amount of 5% to 9% by weight, the fish oil is in an amount of 5% to 20% by weight, and the lemon flavor is in an amount of 0.5% to 5% by weight.

According to an embodiment of the present invention, the colloidal raw material comprises at least three components selected from the group consisting of: xanthan gum, guar gum, gelatin, sodium caseinate, arabic gum, sodium alginate, β-cyclodextrin, sodium carboxymethyl cellulose, Curdlan gum, gelatinized starch, starch acetate, distarch phosphate, phosphorylated distarch phosphate, and methyl cellulose.

According to an embodiment of the present invention, the oily raw material further comprises an ingredient selected from the group consisting of: xylitol, carotene, citric acid, potassium sorbate, sucralose, lemon juice, and RO water at room temperature.

According to an embodiment of the present invention, the stirring in Steps 101 and 102 is uniformly mixed by a high pressure homogenizer at a pressure of 200 to 400 bar.

According to an embodiment of the present invention, the fish oil contains eicosa pentanoic acid (EPA) in an amount of 150 mg/g to 700 mg/g, and docosahexaenoic acid (DHA) in an amount of 100 mg/g to 500 mg/g.

### Example 2

### Analysis of Fish Odor-Free of Fish Odor-Free Fish Oil Composition of Present Invention

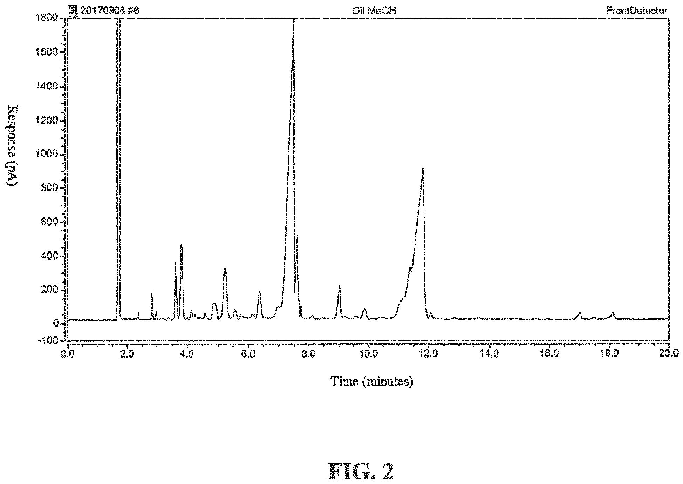

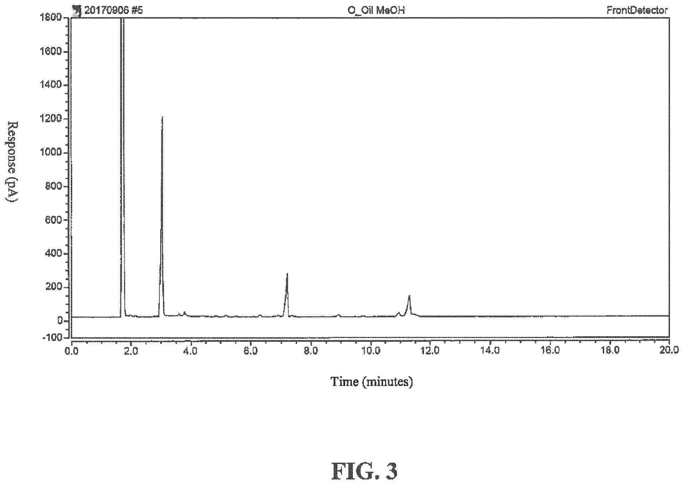

The fish odor-free fish oil composition of the present invention having an EPA content of 150 mg/g and a DHA content of 100 mg/g, and a fish oil capsule product from the DSM Nutritional Products Canada Inc. having an EPA content of 330 mg/g and a DHA content of 220 mg/g were subjected to fish odor-free analysis, respectively. The DB-23 fused silica capillary column obtained from Agilent J&W GC column (length: 30 m; inner diameter: 0.25 mm; film thickness: 0.25 µm; carrier gas: He; collision gas: N₂; injector temperature: 250 °C; FID detector temperature: 260 °C; programming: constant temperature at 215 °C, 35 minutes) was used for detection. The results are shown in FIG. 2 and FIG. 3.

As shown in FIG. 2 and FIG 3, the peak of the fish odor-free fish oil composition of the present invention is much smaller than that of the fish oil capsule product from the DSM Nutritional Products Canada Inc.. In other words, the fish odor of the fish odor-free fish oil composition of the present invention is greatly reduced, and the acceptance of fish oil by the public is improved.

Therefore, the present invention provides a fish odor-free fish oil composition comprising a colloidal raw material and an oily raw material comprising fish oil which can enhance the consumer's acceptance of fish oil supplements, thereby achieving the effects on maintaining elasticity of the artery, lowering cholesterol and triglyceride contents, reducing the incidence of stroke and myocardial infarction, and slowing vision and mental aging.

Although the present invention has been described with reference to the preferred embodiments, it will be apparent to those skilled in the art that a variety of modifications and changes in form and detail may be made without departing from the scope of the present invention defined by the appended claims.

## Claims

1. A fish odor-free fish oil composition, comprising: a colloidal raw material and an oily raw material comprising fish oil.

2. The fish odor-free fish oil composition according to claim 1, wherein the colloidal raw material is in an amount of 5% to 9% by weight, and the fish oil is in an amount of 5% to 20% by weight.

3. The fish odor-free fish oil composition according to claim 1, wherein the oily raw material further comprises a flavor in an amount of 0.5% to 5% by weight.

4. The fish odor-free fish oil composition according to claim 1, wherein the colloidal raw material comprises at least three components selected from the group consisting of: xanthan gum, guar gum, gelatin, sodium caseinate, arabic gum, sodium alginate, β-cyclodextrin, sodium carboxymethyl cellulose, Curdlan gum, gelatinized starch, starch acetate, distarch phosphate, phosphorylated distarch phosphate, and methyl cellulose.

5. The fish odor-free fish oil composition according to claim 3, wherein the flavor is selected from the group consisting of: lemon, mango, peach, orange, passion fruit, grapefruit, and combinations thereof.

6. The fish odor-free fish oil composition according to claim 1, wherein the oily raw material further comprises an ingredient selected from the group consisting of: vitamin E, xylitol, carotene, citric acid, potassium sorbate, sucralose, fruit juice, water, and combinations thereof.

7. A method for the preparation of a fish odor-free fish oil composition, comprising the following steps:
(a) mixing and stirring a colloidal raw material with water and warming to 80 to 85 °C, continuing for 5 to 30 minutes, then cooling to 48 to 53 °C to form a colloidal mixture; and
(b) stirring an oily raw material comprising fish oil and emulsifying in an emulsification tank jointly with the colloidal mixture formed in step (a) to obtain the fish odor-free fish oil composition.

8. The method according to claim 7, wherein the colloidal raw material is in an amount of 5% to 9% by weight, and the fish oil is in an amount of 5% to 20% by weight.

9. The method according to claim 7, wherein the oily raw material further comprises a flavor in an amount of 0.5% to 5% by weight.

10. The method according to claim 7, wherein the colloidal raw material comprises at least three components selected from the group consisting of: xanthan gum, guar gum, gelatin, sodium caseinate, arabic gum, sodium alginate, β-cyclodextrin, sodium carboxymethyl cellulose, Curdlan gum, gelatinized starch, starch acetate, distarch phosphate, phosphorylated distarch phosphate, and methyl cellulose.

11. The method according to claim 7, wherein the flavor is selected from the group consisting of: lemon, mango, peach, orange, passion fruit, grapefruit, and combinations thereof.

12. The method according to claim 7, wherein the oily raw material further comprises an ingredient selected from the group consisting of: vitamin E, xylitol, carotene, citric acid, potassium sorbate, sucralose, fruit juice, water, and combinations thereof.

13. The method according to claim 7, wherein the stirring in the steps (a) and (b) is uniformly mixed by a high pressure homogenizer at a pressure of 200 to 400 bar.
